# EUROPÄISCHE PATENTSCHRIFT

(11) **EP 2 117 623 B1**
(45) Veröffentlichungstag und Bekanntmachung des Hinweises auf die Patenterteilung: **13.11.2013**
(21) Anmeldenummer: 08707588.3
(22) Anmeldetag: 07.02.2008
(51) Int. Cl.: A61M 1/10

(54) **VORRICHTUNG ZUR ÜBERWACHUNG UND OPTIMIERUNG EINES DURCH EINE PUMPE BEWIRKTEN BLUTKREISLAUFS**
APPARATUS FOR MONITORING AND OPTIMIZING BLOOD CIRCULATION GENERATED BY A PUMP
DISPOSITIF PERMETTANT DE CONTRÔLER ET D'OPTIMISER UNE CIRCULATION SANGUINE INDUITE PAR UNE POMPE

(30) Priorität: 09.02.2007 DE 102007007198
(43) Veröffentlichungstag der Anmeldung: 18.11.2009
(73) Patentinhaber: Maquet Cardiopulmonary AG, 76437 Rastatt (DE)
(72) Erfinder: SIMONS, Antoine, P., NL-6228 JD Maastricht (NL)
(74) Vertreter: Kohler Schmid Möbus
(86) Internationale Anmeldenummer: PCT/EP2008/000927
(87) Internationale Veröffentlichungsnummer: WO 2008/095699

(56) Entgegenhaltungen:
- EP-A- 1 354 606
- US-A- 6 066 086
- L. A. BALOA ET AL.: "Control of rotary heart assis devices" PROCEEDINGS OF 2000 AMERICAN CONTROL CONFERENCE (ACC 2000) 28-30 JUNE 2000 CHICAGO, IL, USA, Bd. 5, 28. Juni 2000 (2000-06-28), - 30. Juni 2000 (2000-06-30) Seiten 2982-2986 vol., XP002487700 Proceedings of the 2000 American Control Conference. ACC (IEEE Cat. No.00CH36334) American Autom. Control Council Danvers, MA, USA ISBN: 0-7803-5519-9
- VOLLKRON ET AL.: "Advanced suction detection for an axial flow pump." ARTIFICIAL ORGANS SEP 2006, Bd. 30, Nr. 9, September 2006 (2006-09), Seiten 665-670, XP002487701 ISSN: 0160-564X

## Beschreibung

Die vorliegende Erfindung betrifft eine Vorrichtung um den Blutfluss einer Pumpe automatisch zu überwachen und zu optimieren. Aus den Regelparametern kann dabei auf den Patientenzustand und eventuelle Probleme im durch die Pumpe bewirkten Blutkreislauf geschlossen werden. Dabei wird sichergestellt, dass jederzeit ein ausreichender Blutfluss stattfindet.

Im Stand der Technik sind Blutpumpen und dazugehörige Steuerungen seit längerem bekannt siehe z.B. US 6066086. Die Aufgabe von Blutpumpen in Verbindung mit dazugehörigen Steuerungen ist es, intra- oder extrakorporal einen. Blutkreislauf sicherzustellen und damit die Pumpfunktion des Patientenherzens zu unterstützen oder zu substituieren. Befristet werden solche Blutpumpen zur Substitution der Herzfunktion während Operationen am Herz oder zur Unterstützung der Herzfunktion als Möglichkeit der Erholung des schwachen Herzens in einem extrakorporalen Kreislauf verwendet. Dabei können je nach Erfordernis außer der Blutpumpe alle für den extrakorporalen Kreislauf üblichen Produkte wie z.B. Kanülen, Katheter, Schläuche und Konnektoren, Reservoirs, Oxygenatoren, Wärmetauscher, Blutkonzentratoren und Dialysatoren, Blasenfallen und Filter eingesetzt werden.

Darüber hinaus sind unbefristete Anwendungen der Herzunterstützung und auch Kunstherzen bekannt, wobei in diesen Fällen die Implantation der Pumpe erfolgt.

In allen diesen Anwendungen von Blutpumpen ist es von höchster Wichtigkeit, zuverlässig einen ausreichenden Blutfluss durch die Pumpe zu bewirken. Vor allem bei länger dauerndem Einsatz der Blutpumpe können der Blutfluss und damit zusammenhängende Parameter wie venöser oder arterieller Blutdruck oder Gasfluss und -mischung beim gleichzeitigen Einsatz von Oxygenatoren nicht manuell überwacht und gegebenenfalls korrigiert werden.

Daher ist vor allem für die Anwendung von ECMO (Extracorporeal Membrane Oxygenation) oder ELS (Extracorporeal Life Support) eine zuverlässige automatische Regelung der Blutpumpe erforderlich, da diese Anwendungen über einen längeren Zeitraum von Tagen bis Wochen auch außerhalb des OP und ohne personalintensive Überwachung erfolgen. Aber auch bei Anwendungen in der Herzchirurgie bietet eine automatische Pumpenregelung höheren Komfort in der Handhabung und erhöhte Sicherheit im Falle von Flussstörungen. Außerdem ist eine solche Regelung auch bei implantierbaren Unterstützungssystemen (Ventricular Assist Devices, VAD's) oder bei einem implantierbaren Kunstherz denkbar.

Im Folgenden wird auf die Faktoren eingegangen, die einen erwünschten Blutfluss beeinträchtigen, so dass die Pumpeinstellungen und eventuell zusätzliche Parameter verändert werden müssen.

Aus der Praxis und aus Baloa et al., Vollkron et al. ist für den venösen Blutrückfluss bekannt, dass bei ungenügendem venösem Rückfluss des Blutes die großen venösen Gefäße und/oder der Vorhof des Herzens (je nachdem aus welchem drainiert wird, der linke oder der rechte Vorhof) kollabieren können und dann gar kein oder nur ein unzureichender Blutfluss möglich ist. Dieser venöse Rückfluss hängt u. a. vom Füllungsstatus und der Lage und Konstruktion der venösen Katheter ab. Bei zu geringem Füllungsstatus oder bei Kontakt der Katheteröffnung mit der Gefäßwand kollabiert das Gefäß und behindert den venösen und damit den gesamten Blutfluss. Dieses Phänomen ist in Verbindung mit Zentrifugalpumpen bekannt, kann aber unabhängig vom Pumpprinzip auftreten. Außerdem sind Beeinträchtigungen des venösen Rückflusses durch Thromben o. ä. oder Abknicken des Schlauches in der venösen Linie denkbar. Wird ein solcher Zustand von unzureichendem venösen Rückfluss erreicht, so kann neben unzureichendem Blutfluss auch Schädigung des Blutes durch Kavitation und Bildung von Gasblasen auftreten. Deshalb muss dieser lebensbedrohliche und patientengefährdende Zustand sicher vermieden werden.

Die Überwachung des Drucks auf der venösen Linie ist nicht ausreichend zur Vermeidung des Kollabierens, weil negative Drücke sowohl im Falle der Kollabierung als auch im betriebssicheren Zustand auftreten können. Außerdem ist der Druck auf der venösen Seite der Blutpumpe in hohem Maße von deren Lage im Verhältnis zum Patienten (hydrostatischer Druck) abhängig und damit beeinflussbar.

Außerdem kann auf diese Weise höchstens ein erreichter Kollaps und nur schwer bereits die Tendenz zur Kollabierung erkannt werden.

In der arteriellen Linie sind als Beeinträchtigung des Blutflusses ausschließlich Hindernisse bzw. Verschlüsse durch beispielsweise Thromben oder Embolien oder Abknicken des Schlauches denkbar. Das Problem des Kollabierens der Gefäßwand besteht wegen des arteriellen Drucks nicht. Die Höhe des arteriellen Drucks ist allein als Maß für die Erkennung von Flussstörungen nicht geeignet, weil ein hoher arterieller Flusswiderstand durch die genannten Strömungshindernisse in der arteriellen Linie oder durch einen hohen Gefäßwiderstand bedingt sein kann.

Im Stand der Technik sind Ansätze offenbart, diese Problematik mathematisch zu charakterisieren. Baloa et al. offenbaren z. B. die Methode, durch schrittweises Erhöhen der Pumpendrehzahl, Messen des jeweiligen Flusses und Differenzierung nach der Pumpendrehzahl die differenzielle Größe DRI (Diminishing Return Index) DRI = dQ/dω zu bilden. Im Idealfall ungehinderten Flusses ist diese Kenngröße eine Konstante. Mit beginnendem Kollaps wird DRI geringer, um beim Kollaps den Wert 0 zu erreichen. Es wird allerdings nicht offenbart, wie ein möglicher Kollaps vermieden oder wie ein bereits bestehender Kollaps behoben werden soll.

Die in manchen auf dem Markt befindlichen VAD's realisierte Methode, in solchen Fällen die Pumpe einfach für einige Sekunden abzuschalten, erscheint durch den plötzlichen totalen Ausfall der Pumpfunktion und damit der Kreislaufunterstützung als zu risikoreich. In diesem Fall muss nämlich das geschwächte Patientenherz ohne einen Anpassungsprozess plötzlich die gesamte Last des Blutkreislaufs übernehmen, was zu seiner Überlastung und damit zu einer zusätzlichen Schädigung führen kann. Außerdem kann beim Zurücksetzen der ursprünglichen Drehzahl der Kollaps jederzeit mit den beschriebenen Folgen wieder auftreten. Wenn stattdessen sicherheitshalber eine geringere Pumpendrehzahl voreingestellt wird, kann der dadurch bewirkte geringere Fluss eventuell stabil aufrechterhalten werden. Allerdings ist es dann ein gegenüber der Grundeinstellung reduzierter Fluss, der nicht die volle beabsichtigte Kreislaufunterstützung bewirken kann. Zudem findet die von Baloa et al. beschriebene Applikation Anwendung bei einem VAD, wobei nicht ein Gefäß, sondern der Ventrikel kollabiert.

Vollkron et al. ermittelt den venösen Rückfluss anhand der Pulsatilität im Flusssignal, was nur bei einem schlagenden Herz gegeben ist. Im Fall einer zu hohen Pumpengeschwindigkeit drosselt man diese langsam bis auf ein Minimum, wonach die Geschwindigkeit langsam wieder hochgefahren wird bis zu einem maximalen sicheren oder gewünschten Fluss. Dieses langsame Herunter- und Hochfahren bedarf jedoch einer gewissen Zeitspanne, in der die Pumpe den optimierten Fluss sucht, jedoch in der eine optimale Unterstützung nicht gewährleistet ist.

Vor diesem Hintergrund liegt der vorliegenden Erfindung die Aufgabe zugrunde, eine Vorrichtung zur Durchführung eines Verfahrens bereitzustellen, den Blutfluss von Blutpumpen unter Vermeidung lebensbedrohlicher Zustände automatisch zu regeln und im Rahmen der gewünschten Vorgaben zu optimieren, wobei das Verfahren universell anwendbar sein sollte, d. h. auch bei einem Herzstillstand sicher funktionieren und es sollten im Fall ausgeregelter Beeinträchtigungen des Blutflusses deren Lage im Kreislauf, venös oder arteriell, erkannt werden können, um gezielt rasche Maßnahmen ergreifen zu können.

Diese Aufgabe wird durch die erfindungsgemäße Vorrichtung wie beansprucht vollkommen gelöst.

Dieses neue Verfahren kann automatisch periodische Eingriffe in die Pumpendrehzahl vornehmen, wobei über die Bestimmung des Effektes auf den Fluss durch Aufnahme der Messwerte von Pumpenparametern wie der Drehzahl und des Blutflusses pro Zeiteinheit und deren Verhältnis zueinander differentielle Kenngrößen ermittelt werden, die die Fließzustände charakterisieren und zur Erfassung und Behebung von Rückflussstörungen und zur Analyse des Herzzustandes geeignet sind.

Der je nach Patientenbedarf benötigte Blutfluss wird in bekannter Weise vom behandelnden Arzt oder Kardiotechniker festgelegt und damit ist auch die für das erfindungsgemäße Verfahren benötigte Zahlengröße "3/4 des im Kreislauf benötigten Blutflusses" bekannt. Beispielsweise lässt sich der benötigte Blutfluss über die Formel von du Bois für eine patientenbezogene Körperoberfläche berechnen, wobei Cardiac Indices im Bereich von 2,5 - 4 l min⁻¹ m⁻² normal sind.

Die Pumpendrehzahl wird im Gegensatz zu Baloa et al. nicht nur hoch-, sondern auch heruntergeregelt und der veränderte Blutfluss gemessen. Auf diese Weise kann bei erkannter Tendenz zu kollabieren durch Reduktion der Pumpendrehzahl wieder ein ausreichender venöser Rückfluss mit Rückbildung des beginnenden Kollapses erzielt werden. Danach kann schrittweise durch Erhöhung der Drehzahl und Messung des veränderten Flusses (Blutfluss) die Drehzahl und der Fluss optimiert werden, wobei stabile Werte oberhalb des Ausgangswertes für beginnenden Kollaps erzielt werden können.

Das Verfahren, einen beginnenden Kollaps nicht nur zu erkennen, sondern auch rasch zu beheben und danach den Fluss zu optimieren, unterscheidet sich deutlich vom Stand der Technik (Baloa et al., Vollkron et al.), wo zwar auch differenzielle Werte ermittelt werden, aber keine Lösung zur Rückbildung eines beginnenden oder vorhandenen Kollapses offenbart wird. Die in manchen auf dem Markt befindlichen VAD's realisierte Methode, in solchen Fällen die Pumpe einfach für einige Sekunden abzuschalten, erscheint durch den plötzlichen totalen Ausfall der Pumpfunktion und damit der Kreislaufunterstützung als zu risikoreich. In diesem Falle muss nämlich das geschwächte Patientenherz ohne einen Anpassungsprozess plötzlich die gesamte Last des Blutkreislaufs übernehmen, was zu seiner Überlastung und damit zu einer zusätzlichen Schädigung führen kann.

Andererseits bedarf die von Vollkron et al. offenbarte Behebung eines Kollapses einer gewissen Zeit, bis die Pumpengeschwindigkeit einmal gedrosselt wurde, und auch anschließend wieder hochgefahren wurde. Vor allem hier unterscheidet sich das erfindungsgemäße Prinzip durch eine rapide Drosselung der Pumpe mit einem darauf folgenden Pumpengeschwindigkeitsanstieg, wobei diese Funktion auch bei nicht pulsatilem Blutfluss, z. B. bei Herzstillstand, gegeben ist.

Die erfindungsgemäße Vorrichtung wird anhand der nachfolgenden Figuren der Zeichnung beschrieben.

Es zeigt:
- Fig. 1: ein Schema eines minimierten extrakorporalen Bypasses mit Pumpe, Oxygenator und Schlauchlinien;
- Fig. 2: ein Flussdiagramm mit der Illustration der Hauptschritte des Algorithmus;
- Fig. 3: ein Schema des erfindungsgemäßen Regelkreises, wobei der Sollwert ein Bereich von erlaubten Werten der Differential-Flow-Speed-Ratio (DFSR) ist, welcher es der Regelung ermöglicht, die Pumpengeschwindigkeit (PS) zu steuern und demzufolge einen Fluss erzeugt, wobei ein maximaler Fluss auch die DFSR-Regelung beeinflussen, d. h. eine Überperfusion verhindern kann;
- Fig. 4: einen grafischen Zusammenhang zwischen veränderter Pumpleistung und ermittelten DFSR-Werten; und
- Fig. 5: eine in einem Schaubild dargestellte Regelungsabfolge, wie sie erfindungsgemäß bevorzugt erfolgen kann.

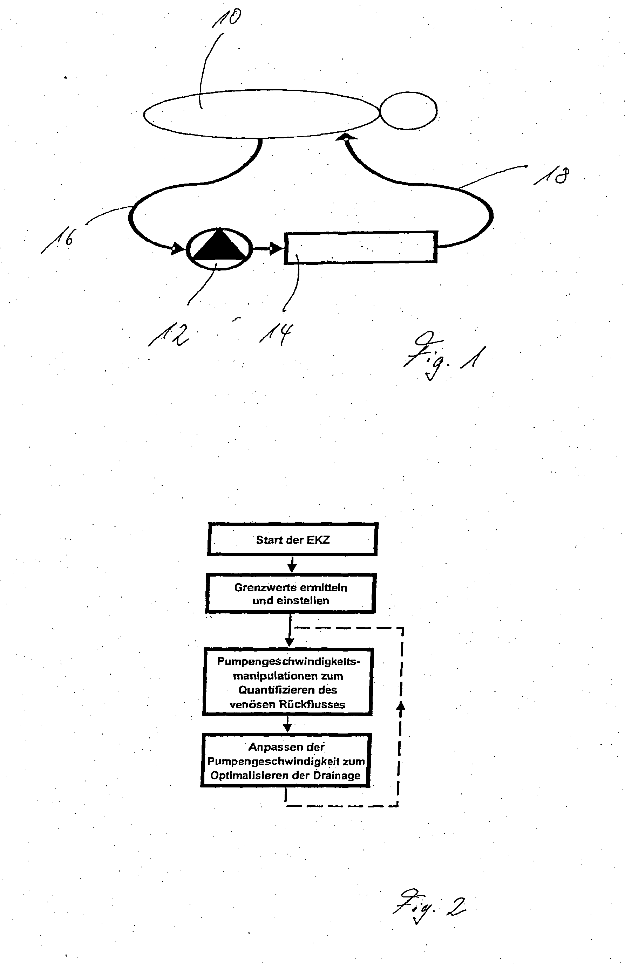

In Fig. 1 ist mit 10 ein Patient angedeutet, der an einen extrakorporalen Blutkreislauf, bestehend aus einer Pumpe 12, einem Oxygenator 14, einer venösen Schlauchlinie 16 und einer arteriellen Schlauchlinie 18, angeschlossen ist.

Während der Anfangsphase eines minimierten geschlossenen extrakorporalen Kreislaufsystems (Fig. 1) wird die Pumpendrehzahl (PS) schrittweise um α U/min erhöht. Dabei wird ständig über Δ_{Fluss}/Δ_{Drehzahl} (= Δflow/ΔPS [I/min.] [Umdrehung/min]) der Wert "Differential-Flow-Speed-Ratio" (DFSR) berechnet, bis ¾ des benötigten Flusses erreicht wird. Der Mittelwert der berechneten Differentiale stellt einen angenäherten Wert des Fluss-Umdrehuhgszahl-Verhältnisses für jeden einzelnen Patient mit Kreislaufunterstützung dar. Dieser Wert wird halbiert und als Schwellenwert T benutzt (definiert). Während des Bypasses wird die Pumpendrehzahl (Pump Speed, PS) um β U/min während ε Sekunden reduziert. Nach der Pumpendrehzahlreduzierung (dPS) wird die Flussänderung (dF) berechnet und daraus die differentielle Größe Differential-Flow-Speed-Ratio (DFSR), d. h. Δflow/ΔPS ermittelt.

DFSR ≤ 1T bedeutet eine übermäßige Dränage, also einen zu geringen Rückfluss, und erfordert eine plötzliche Abregelung der Pumpendrehzahl PS auf 50% der anfänglichen Pumpendrehzahl. Nach γ Sekunden steigt PS schrittweise um δ U/min auf bis zu 95% der anfänglichen Pumpendrehzahl PS an. DFSR>2T bedeutet zu geringe Dränage, was einen Anstieg der Pumpengeschwindigkeit PS um ε U/min erfordert. Die Berechnung des neuen DFSR zeigt den Erfolg der Rückflussoptimierung an.

Der Wert von 1,5T wird als oberer Schwellwert angesetzt. DFSR> 1,5T zeigt an, dass eine höhere Pumpendrehzahl und damit auch ein höherer Fluss möglich ist. Bei Aktivierung der Option kann DFSR> 1,5T die Pumpe veranlassen, die Drehzahl um ζ U/min zu erhöhen. Die Berechnung des neuen DFSR zeigt den Erfolg der Rückflussoptimierung an. Wenn man eine mögliche Überperfusion vermeiden möchte, kann ein maximaler Fluss eingegeben werden, der dann die Priorität gegenüber der Drehzahlerhöhung erhält.

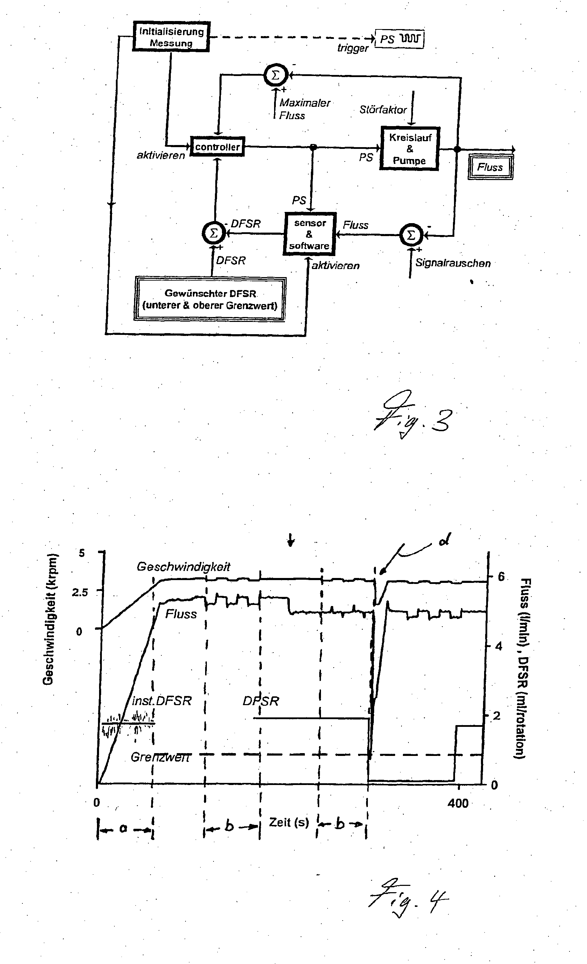

Ein Schema der oben beschriebenen Kontrolleinheit ist in Fig. 2 und 3 abgebildet. Fig. 4 zeigt Ergebnisse einer praktischen Anwendung.

Fig. 2 zeigt in einem Flussdiagramm die erfindungsgemäße Vorgehensweise (Algorithmus) zur Erreichung einer optimierten Drainage. Nach dem Start des extrakorporalen Blutkreislaufs (EKZ) werden die notwendigen patientenbezogenen Grenzwerte ermittelt, wie zu Fig. 1 beispielhaft beschrieben. Anschließend wird über eine Veränderung der Pumpenleistung der venöse Blutrückfluss quantifiziert und daraus die Pumpenleistung ermittelt und eingestellt, die eine optimale Drainage ermöglicht.

Fig. 3 stellt einen erfindungsgemäßen Regelkreis dar, der über gewünschte DFSR-Werte ständig neue DFSR-Istwerte aus dem augenblicklichen Fluss der Pumpendrehzahl ermittelt, die DFSR-Istwerte mit den gewünschten DFSR-Werten abgleicht und daraus ständig die Pumpendrehzahl neu einstellt, um sowohl eine übermäßige als auch eine zu geringe Drainage zu verhindern.

Fig. 4 veranschaulicht wie nach dem Hochfahren der Pumpe und gleichzeitigem Messen der DFSR ein Mittelwert gebildet (inst. DFSR) wird. Die Hälfte dieses Wertes wird angegeben als Grenzwert. Nach Ermitteln des DFSR wird der venöse Rückfluss reduziert (dargestellt mit dem Pfeil). Eine folgende Messung zur Ermittlung des DFSR stellt eine Reduktion desselben fest, wonach die Pumpengeschwindigkeit mittels einer 2-Step-Reduzierung effektiv auf 95% gedrosselt wird. Anschließende Messung des DFSR zeigt deutlich eine Steigung dieses Wertes, und eine Optimierung der Drainage, sprich Anpassung der Pumpengeschwindigkeit an den zur Verfügung stehenden venösen Fluss.

Weiterhin sind in die Fig. 4 die Verfahrensschritte a und b aus dem Verfahrensanspruch 1 eingezeichnet, wobei der Verfahrensschritt d mit einem weiteren Pfeil angezeigt ist.

Rückflusshindernisse erhöhen den Flusswiderstand auf der Einlassseite der Pumpe, was die Pumpenvorlast reduziert und den Pumpfluss negativ beeinflusst. Im Falle von Clotting eines Oxygenators ist der Widerstand gleichfalls erhöht, aber dieser zusätzliche Widerstand tritt dann hinter der Pumpe auf. Beide Fälle beeinflussen DFSR, aber zur Unterscheidung zwischen Clotting des Oxygenators und Behinderung des venösen Rückflusses zur Pumpe kann die zusätzliche Messung des Druckabfalls über den Oxygenator (Druck am Einlass - Druck am Auslass) die benötigte Information liefern. Wenn DFSR abfällt, zeigt die Höhe des Differenzdrucks am Oxygenator die Ursache des gestiegenen Widerstands an, d. h. ob sie vor oder nach der Pumpe liegt. Falls der Differenzdruck gering ist, liegt eine Behinderung des venösen Rückflusses vor, bei hohem Differenzdruck ist Clotting des Oxygenators eingetreten.

Des Weiteren liefert die Messung von DFSR Informationen über die Pumpkapazität des Herzens, insbesondere der rechten Herzkammer. Im Fall eines sich erholenden Herzens und einer dadurch verringerten durchschnittlichen Pumpendrehzahl sollte sich DFSR nicht ändern. Das erholte Herz reagiert nach dem Frank-Starling-Mechanismus auf die angestiegene Vorlast und versucht, die benötigte Ausstoßleistung zu erzielen. Daher bleiben venöser und arterieller Druck konstant, und damit DFSR. Bei einem insuffizienten oder nur teilweise erholten Herzen führt eine Reduzierung der mittleren Pumpendrehzahl zu einer Erhöhung von DFSR. Das insuffiziente Herz ist nicht fähig, die erhöhte Vorlast zu bewältigen, wenn die Pumpenunterstützung verringert wird. Daher steigt in diesem Fall der venöse Druck und der arterielle Druck verringert sich. Der Körper wiederum reagiert auf den abfallenden arteriellen Druck mit Vasokonstriktion, um ein weiteres Abfallen des arteriellen Drucks zu kompensieren. Dadurch steigt die Vorlast der Pumpe, während die Nachlast näherungsweise konstant bleibt: DFSR steigt an.

Die Reaktion auf drehzahlreduzierende Regelschritte kann in Kombination mit einer Flussmusteranalyse dazu verwendet werden, den Herzstatus zu ermitteln. Das schlagende Herz beeinflusst das Flussmuster einer unterstützenden Zentrifugalpumpe derart, dass während der Systole der Aortendruck ansteigt, und dabei der Fluss über die Pumpe abnimmt. Wenn die mittlere Pumpendrehzahl verringert wird und daraufhin drehzahlreduzierende Regelschritte folgen, geben die nachfolgenden Pulse des Flusssignals wertvolle Informationen über den Herzstatus und dessen Pumpreserve. Nach der Reduzierung der Unterstützung gibt die Steilheit und die Amplitude der Flusspulse direkte Informationen über die Kontraktionsfähigkeit und damit die Regenerierung des Herzens.

Die obigen Methoden können dazu verwendet werden, den Herzstatus und die Reserven der Pumpfähigkeit zu bestimmen, und können sinnvoll dazu verwendet werden, den Patienten von der Herzunterstützung zu entwöhnen. Sie lassen sich sehr hilfreich als Werkzeug für das Herzmonitoring anwenden, während dieses über periodische Drehzahlreduzierungen mehr Vorlast erhält und dabei trainiert wird.

Eine andere Möglichkeit, Herzstatus und Herzregeneration zu ermitteln, ist, die Zeit zu bestimmen, die das Herz benötigt, die abrupte Pumpunterstützung zu kompensieren, und den vom Körper benötigten Gesamtfluss, bestehend aus Pumpenfluss und Herzauswurfleistung, zu liefern.

In beiden Methoden wird direkt der Frank-Starling-Mechanismus ausgenutzt. Diese Anwendung benötigt aber, im Unterschied zur nichtinvasiven Bestimmung des DFSR, invasive Messung des arteriellen und venösen Blutdrucks.

Auf diese Weise ist es also möglich, die Überwachung und die Optimierung des Blutflusses zu automatisieren, was bei einer Langzeitanwendung zur Herzunterstützung und -regenerierung notwendig ist. Darüber hinaus lassen sich Hinweise über die Erholung des Herzens über die Reaktion des Herzens auf einen verminderten Blutfluss über die Pumpe ermitteln. Je höher die Pulsation im arteriellen Blutdruck bei Verringerung der Pumpleistung hervortritt, desto höher ist die Leistung und damit die Erholung des Herzens. Es kann also während der Zeit der Herzunterstützung die erforderliche Unterstützung automatisch gegeben werden, wobei im Falle der Verringerung der Pumpleistung des Herzens automatisch die Pumpe einen größeren Teil der Pumpleistung übernehmen kann. Auf diese Weise ist eine risikofreie Regenerierung des Herzens und danach eine automatische stufenlose Entwöhnung von der Pumpe möglich.

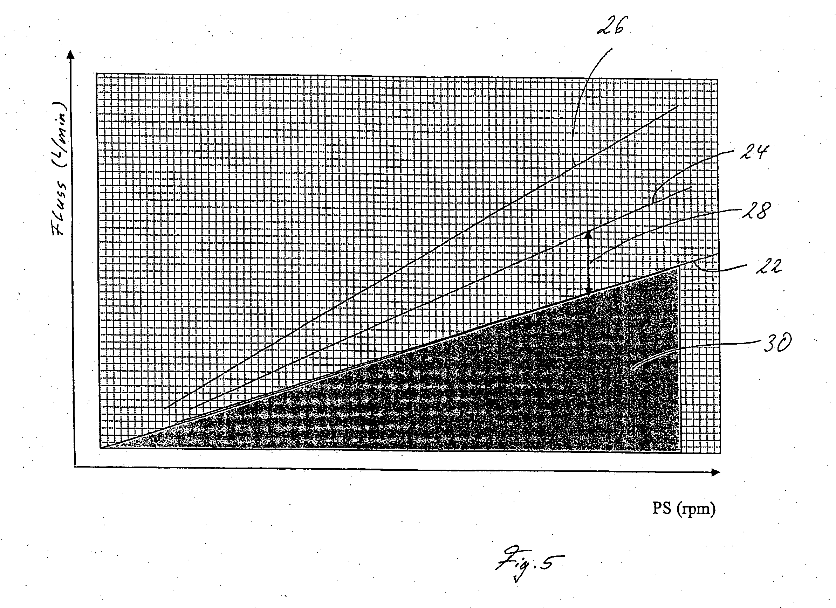

Fig. 5 zeigt beispielhaft, wie eine erfindungsgemäße Regelung erfolgen kann. Nach Ermittlung des benötigten Blutflusses, die dem hier angesprochenen Fachmann geläufig ist, startet das Regelverfahren und ermittelt DFSR. Ein Bruchteil davon, hier im Beispiel die Hälfte davon, wird wie beschrieben als Schwellenwert T definiert.

In der Fig. 5 ist mit 22 die Gerade für DFSR = 1T dargestellt. Mit dem Bezugszeichen 24 ist der Verlauf von DFSR = 1,5T gezeigt und DFSR = 2T ist mit 26 gekennzeichnet. Zwischen den Geraden 22 und 24 ist der Betriebsbereich 28 des erfindungsgemäßen Verfahrens angegeben. Unterhalb der Geraden 22 (DFSR = 1T) ist als grauer Bereich 30 (DFSR < 1T) der nicht gewünschte Betriebsbereich markiert, in dem teilweise oder vollständige Kollabierung auftreten kann. Dieser Bereich ist kritisch für die Patientensicherheit und muss deshalb unbedingt sicher vermieden werden.

Bei DFSR ≥ 1,5T wird die Pumpendrehzahl erhöht, bei DFSR ≤ 1T erniedrigt (und nachfolgend wieder erhöht). Damit wird ein stabiler Betrieb im Betriebsbereich zwischen 1T und 1,5T erzielt.

Die Startpunkte der Geraden DFSR = 1T, DFSR = 1,5T und DFSR = 2T beginnen im obigen Beispieldiagramm nicht im Ursprung. Es versteht sich, dass je nach vorliegenden Druckverhältnissen im Einsatz bei einer beispielhaft aufgeführten Zentrifugalpumpe ein durch diese Pumpe bewirkter positiver Fluss > 0 erst bei einer gegebenen Drehzahl PS bewirkt werden kann und die Ermittlung von DFSR somit erst mit dieser Drehzahl beginnen kann und bei ¾ des gewünschten Flusses endet. Daher sind zur Ermittlung von DFSR und damit von T die im Pumpkreislauf auftretenden Werte für Drehzahl (PS) und durch die Pumpe bewirkten (= positiven) Fluss > 0 bis ¾ des gewünschten Flusses auszuwerten.

Zum allgemeinen Verständnis wird nochmals darauf hingewiesen, dass DFSR über die Größen ΔFluss/ΔDrehzahl für eine Abfolge verschiedener Drehzahlen gebildet wird. Somit ist dieser Wert aber als differenzielle Größe zu verstehen. Daher stellt DFSR die 1. Ableitung (Steigung der Funktion ΔFluss/ΔDrehzahl an jedem Punkt) der jeweiligen Funktion dar.

Für sich nicht verändernde Rückflussverhältnisse, also bei nahezu idealem, ungehindertem Blutfluss, ist für diese Funktion eine Gerade zu erwarten und daraus als Ableitung eine konstante Größe DFSR. Dieses Verhalten wird zur Ermittlung des inst. DFSR und damit des Schwellenwertes T ausgenutzt.

Eine Änderung dieser Steigung, gleich bedeutend für einen veränderten Wert für DFSR im betrachteten Bereich der Pumpendrehzahl, deutet auf eine Veränderung der Flussbedingungen hin.

In dem erfindungsgemäßen Verfahren werden von einer gegebenen Pumpendrehzahl aus periodische Drehzahlinterventionen durchgeführt und aus ΔFluss/ΔDrehzahl für obere und untere betrachtete Drehzahlen der Intervention die differentielle Größe DFSR für den Bereich dieser Intervention gebildet.

Durch einen Vergleich mit dem Schwellenwert I (Bruchteil von inst. DFSR, im speziellen Fall ½ inst. DFSR) wird die Pumpendrehzahl derart geregelt, dass Flussverhältnisse innerhalb eines vorgegebenen Bereichs für DFSR eingehalten werden. Dieser vorgegebene Bereich von T bis 1,5T charakterisiert in diesem Ausführungsbeispiel an das verfügbare Rückflussvolumen angepasste Pumpendrehzahlen, wobei einerseits Überdrainage vermieden wird und andererseits der vorgegebene benötigte Blutfluss unter variablen Drainagebedingungen bestmöglich erreicht wird.

Es handelt sich also nicht um ein Regelverfahren für den Blutfluss im Allgemeinen, sondern um das Anpassen der Pumpendrehzahl an die Flussverhältnisse mit dem vorgegebenen Zielwert für den maximalen Blutfluss.

In einer Weiterbildung dieses Verfahrens kann die Gaszufuhr eines im Blutkreislauf befindlichen Oxygenators in den Regelkreis integriert werden. Da der Blutfluss von der Pumpenregelung variabel eingeregelt wird, muss auch der Gasfluss und der Sauerstoffgehalt der Gaszufuhr dem jeweiligen Blutfluss und den Patienten-Erfordernissen automatisch angepasst werden. Dazu wird vorgeschlagen, den vorhandenen Blutfluss und die online gemessenen Partialdrücke der Blutgase pO₂ und pCO₂ zur Regelung der Gaszufuhr zu verwenden. Sind beide Partialdrucke normal, so kann die Gasmischung und der Gasfluss beibehalten werden. Ist pO₂ zu gering, so ist der Sauerstoffgehalt des zugeführten Gasgemisches automatisch zu erhöhen und umgekehrt. Ein zu hoher Wert von pCO₂ wird dagegen durch eine Erhöhung des Gasflusses, ein zu niedriger dagegen durch die Verringerung des Gasflusses eingeregelt. Der Algorithmus kann analog dem für den Blutfluss vorgeschlagenen mit Schwell- oder Grenzwerten ausgestaltet sein.

In diesem Falle ist die automatische Erfassung des Differenzdruckes am Oxygenator und eine Warnmeldung bei Erreichen eines Schwellwertes für Differenzdruck oder Gaspartialdrücke von besonderer Bedeutung. Dadurch kann sowohl die Gefahr einer beginnenden Unterversorgung des Patienten an Herz- wie an Lungenfunktion erkannt und durch entsprechende Maßnahmen, wie z. B. den Tausch eines verclotteten Oxygenators, behoben werden.

Auch wenn in Fig. 4 nur Messwerte von Zentrifugalpumpen offenbart wurden, ist für den Fachmann offensichtlich, dass die Problematik des venösen Rückflusses und der Gefahr des Kollabierens von Gefäßen oder Ventrikeln prinzipiell bei Anwendung von Blutpumpen jeglicher Funktionsweise auftreten und erfindungsgemäß gelöst werden kann.

Es versteht sich, dass bei den obigen nichtinvasiven Verfahren als Parameter nicht nur der angegebene Fluss der Pumpe, sondern auch die diesem Fluss proportionale Kenngrößen der Pumpe, wie z. B. die Leistungs- bzw. Stromaufnahme einer Zentrifugalpumpe oder der Strom bzw. die Leistungsaufnahme aktiver Magnetlager von Axialpumpen als Regelparameter verwendet werden können, ohne den Rahmen dieser Erfindung zu verlassen.

In einem Verfahren zur automatischen Regelung von Blutpumpen wird durch periodische Drehzahlinterventionen und dabei auftretende Flussänderungen über eine gebildete differentielle Größe und einen Regelalgorithmus eine Optimierung des Blutflusses erreicht. Zusätzlich kann die Lage von möglichen Flusswiderständen auf venöser oder arterieller Seite ermittelt werden.

Verfahren zur Regelung von Blutpumpen, wobei durch automatische periodische Drehzahl- bzw. Pumpgeschwindigkeitsveränderungen und der dazu gemessenen Veränderung der Förderleistung der Pumpe die differenzielle Kenngröße Differential-Flow-Speed-Ratio (DFSR) nach Δ_{Förderleistung}/Δ_{Drehzahl/Pumpgeschwindigkeit} gebildet wird, deren Höhe und Verlauf in einem Algorithmus benutzt wird, der die Optimierung des Blutflusses bewirkt.

Verfahren, wobei die automatischen periodischen Drehzahl- bzw. Pumpgeschwindigkeitsveränderungen sowohl Erhöhungen als auch Erniedrigungen sein können.

Verfahren, wobei die Veränderung der Förderleistung über Veränderung von Pumpenparametern bestimmt wird.

Verfahren, wobei die Ermittlung der Drehzahl- bzw. Pumpgeschwindigkeitsveränderungen und der dazu gemessenen Veränderung der Förderleistung der Pumpe nicht invasiv erfolgt.

Verfahren, wobei die Ermittlung mindestens eines Parameters invasiv erfolgt.

Medizinische Vorrichtung, die eine Pumpe zur Förderung von Blut mit einer Regelung enthält.

## Patentansprüche

1. Vorrichtung zum Betreiben von Blutpumpen innerhalb eines intra- oder extrakorporalen Blutkreislaufs, **dadurch gekennzeichnet, dass** mit ihr
a) ein Schwellenwert T ermittelbar ist, der ein Teil eines Mittelwerts aus gemessenen Kenngrößen DFSR ist, wobei die einzelnen DFSR-Kenngrößen aus dem Quotienten einer differenziellen Blutförderleistung zu einer dazu gemessenen differenziellen Blutpumpendrehzahl errechnet werden, wobei schrittweise die Blutpumpendrehzahl bis ¾ des im Kreislauf benötigten Blutflusses erhöhbar ist und für jeden Veränderungsschritt eine DFSR-Kenngröße berechenbar ist;
b) die Blutpumpendrehzahl reduzierbar ist und schrittweise die dazugehörigen DFSR-Kenngrößen berechenbar sind;
c) die DFSR-Kenngrößen aus b) mit dem Schwellenwert T vergleichbar sind;
d) die Blutpumpendrehzahl je nachdem ob die DFSR-Kenngröße nach b) größer oder kleiner als der Schwellenwert T ist, veränderbar ist.

2. Vorrichtung nach Anspruch 1, **dadurch gekennzeichnet, dass** zum Betreiben der Blutpumpen der Schwellenwert T die Hälfte des Mittelwerts aus gemessenen Kenngrößen DFSR ist, die bis zum Erreichen von ¾ des im Kreislauf benötigten Blutflusses erfassbar sind.

3. Vorrichtung nach Anspruch 1 oder 2, **dadurch gekennzeichnet, dass** bei DFSR ≤ 1T die Blutpumpendrehzahl reduzierbar ist und nachfolgend über eine definierte Zeit wieder erhöhbar ist.

4. Vorrichtung nach einem der Ansprüche 1 bis 3, **dadurch gekennzeichnet, dass** bei DSFR ≥ 1,5T die Blutpumpendrehzahl erhöhbar ist.

5. Vorrichtung zum Betreiben von Blutpumpen innerhalb eines intra- oder extrakorporalen Blutkreislaufs nach einem der Ansprüche 1 bis 4, **dadurch gekennzeichnet, dass** der Blutpumpenkreislauf erste Mittel zur Erfassung, Berechnung und zum Vergleich von ermittelten DFSR-Kenngrößen aufweist und zweite Mittel der Vorrichtung zur Verstellung der Blutpumpendrehzahl in Abhängigkeit von einem Ergebnis aus einem Vergleich von erfassten DFSR-Kenngrößen mit Schwellenwerten T vorgesehen sind.

## Claims

1. A device for operating blood pumps within an intra- or extracorporeal blood circulation, **characterized in that** with said device the following steps can be carried out:
a) a threshold value T can be determined, which is a part of a mean value of measured characteristics DFSR, the individual DFSR characteristics being calculated from the quotient of a differential blood flow rate relative to a differential blood pump speed measured for this purpose, in which case the blood pump speed can be increased step by step up to ¾ of the blood flow required in the circulation, and a DFSR characteristic can be calculated for each step of change;
b) the blood pump speed can be reduced and step by step the associated DFSR characteristics can be calculated;
c) the DFSR characteristics from b) can be compared with the threshold value T;
d) the blood pump speed can be changed dependent upon whether the DFSR characteristic according to b) is greater or smaller than the threshold value T.

2. A device according to Claim 1, **characterized in that**, for operating the blood pumps, the threshold value T is half of the mean value of measured characteristics DFSR detectable up to achieving ¾ of the blood flow required in the circulation.

3. A device according to Claim 1 or 2, **characterized in that** when DFSR ≤ 1T, the blood pump speed can be reduced and then increased again over a specific period of time.

4. A device according to one of Claims 1 to 3, **characterized in that** when DFSR, ≥ 1.5T, the blood pump speed can be increased.

5. A device for operating blood pumps within an intra- or extracorporeal blood circulation according to one of Claims 1 to 4, **characterized in that** the blood pump circuit comprises first means for detecting, calculating and comparing determined DFSR characteristics, and second means of the device are provided for adjusting the blood pump speed as a function of a result obtained from a comparison of detected DFSR characteristics with threshold values T.

## Revendications

1. Dispositif destiné à faire fonctionner des pompes à sang au sein d'une circulation sanguine intracorporelle ou extracorporelle, **caractérisé en ce qu'**avec lui
a) une valeur seuil T peut être déterminée, laquelle représente une partie d'une valeur moyenne obtenue à partir de caractéristiques mesurées DFSR, les différentes caractéristiques DFSR étant calculées à partir du quotient d'un débit sanguin différentiel sur un régime de pompe à sang différentiel mesuré à cette fin, le régime de pompe à sang pouvant être augmenté progressivement jusqu'au ¾ du flux sanguin requis dans la circulation et une caractéristique DFSR étant calculable pour chaque étape de changement ;
b) le régime de pompe à sang peut être réduit et les caractéristiques DFSR qui s'y rapportent peuvent être calculées progressivement ;
c) les caractéristiques DFSR obtenues en b) sont comparables à la valeur seuil T ;
d) le régime de pompe à sang est modifiable selon que la caractéristique DFSR selon b) est supérieure ou inférieure à la valeur seuil T.

2. Dispositif selon la revendication 1, **caractérisé en ce que**, pour faire fonctionner les pompes à sang, la valeur seuil T correspond à la moitié de la valeur moyenne obtenue à partir de caractéristiques mesurées DFSR, qui sont saisissables jusqu'à l'atteinte des ¾ du flux sanguin requis dans la circulation.

3. Dispositif selon la revendication 1 ou 2, **caractérisé en ce que** le régime de pompe à sang peut être réduit pour DFSR ≤ 1 T et il peut être de nouveau augmenté par la suite sur un temps défini.

4. Dispositif selon l'une quelconque des revendications 1 à 3, **caractérisé en ce que** le régime de pompe à sang peut être augmenté pour DFSR ≥ 1,5T.

5. Dispositif destiné à faire fonctionner des pompes à sang au sein d'une circulation sanguine intracorporelle ou extracorporelle selon l'une quelconque des revendications 1 à 4, **caractérisé en ce que** le circuit de pompe à sang présente des premiers moyens permettant de saisir, calculer et comparer des caractéristiques DFSR déterminées et des seconds moyens du dispositif sont prévus en vue de réguler le régime de pompe à sang en fonction d'un résultat obtenu par comparaison de caractéristiques DFSR saisies avec des valeurs seuils T.
